# EUROPEAN PATENT APPLICATION

(11) **EP 1 886 707 A1**
(43) Date of publication of application: **13.02.2008**
(21) Application number: 06254214.7
(22) Date of filing: 10.08.2006
(51) Int. Cl.: A61M 21/00

(54) **Sleep enhancing device**

(71) Applicant: Future Acoustic LLP, London SW7 2EU (GB)
(72) Inventor: Raptopoulos, Andreas, London, SE1 9LF (GB); Barnardo, Christopher John Andrew, Hertfordshire, CM23 5JQ (GB); Rothwell, Nicholas, London, SE16 5NY (GB)
(74) Representative: Addison, Ann Bridget

(57) **Abstract**

The present invention provides a sleep enhancing device and a corresponding method for sleep enhancement. The device comprises a transducer (14) for detecting ambient noise in a sleeping environment and for generating electrical signals representing the ambient noise, and means (40) responsive to a state of sleep of a user in the sleeping environment for providing a corresponding control input. A processor (46) serves for analysing the electrical signals to evaluate the ambient noise and provide an evaluation output. A sound bank (54) stores audio data representing soothing sounds, and sound generating means responsive to the control input and the evaluation output selects audio data from the sound bank and provides an audio output. Output means (38) output the audio output as a sound output.

## Description

This invention concerns a sleep enhancing device for monitoring noises in a sleeping environment and for generating a calming or soothing sound output to mask noise intrusion and encourage sleep.

Sound screening systems are known for monitoring and regulating sound in an environmental space, such systems detecting the ambient noise and generating masking sound or tonal sequences to generate pleasing sound in the environmental space. In particular, sound screening systems are described in our earlier published International Patent Applications Nos. WO 01/37256 and WO 02/25631 and published US Patent Application No. 10/996330, all of which are incorporated herein by reference.

These known arrangements are designed to regulate the sound in an environmental space in dependence upon the nature and level of the ambient noise, and in response to a pre-selected control input made by a user according to taste or certain environmental conditions.

A study carried out by the Centre for Occupational and Health Psychology, Cardiff University, and the Psychopharmacology Unit, University of Bristol, has also considered the links between noise and insomnia. The research described in this report was supported by the UK Department of Health and Department of Environment, and the full report may be found at:
http://www.dh.gov.uk/assetRoot/04/03/42/61/04034261.pdf
The study looked in particular at the type of noise that respondents cited as disturbing, and the type of noise that they felt prevented them from falling asleep or woke them once they were asleep. It was noted from the study that people are most likely to feel tired and/or irritable as the result of disturbing or unwanted and intrusive noise, and that the type of noise that predominantly intrudes at bedtime is that of traffic, noisy neighbours and other people's music.

Other studies relating to physiological processes and factors influencing such processes are described in US Patent 5267942, and the following publications:
a) Y. Shiihara et al, Continuous recordings of skin conductance change during sleep. Psychiatry and Clinical neurosciences (2000), 54, 268-269
b) M W Johns et al, Monitoring of hospital patients by measurement of electrical resistance of skin. Journal of applied physiology, Vol 27, No. 6, December 1969
**c)** A W Kaszniak, Psychology of Consciousness (Psyc. 358), Class Notes Sping 2002*.*

It is an aim of the present invention to provide a sleep enhancing device for monitoring sound in a sleeping environment and for generating a calming or masking sound output in response to intrusive noises.

It is another aim of the present invention to provide a sleep enhancing device for generating a calming or masking sound output according to a current state of sleep of an individual in a sleeping environment.

It is another aim of the present invention to provide a device for detecting and evaluating ambient noise in a sleeping environment and for generating a soothing sound in dependence upon the outcome of the evaluation and in dependence upon a state of sleep of an individual in the sleeping environment.

It is to be noted that the expression "a state of sleep" as used herein includes also waking states associated with sleeping, such as a pre-sleep state.

In order best to appreciate the present invention, it is necessary to understand something about the human sleep pattern, which involves 5 stages of sleep, as indicated below.

### The stages of sleep

As a person falls asleep, they enter transition sleep, called Stage 1, and begin a first "sleep cycle", usually lasting about 90 minutes. Within a few minutes, this transition sleep evolves into "baseline" sleep, called Stage 2. Stage 2 sleep occupies approximately 50-65% of overall sleeping time. Within 15-20 minutes, the baseline sleep slowly evolves into Stage 3 then Stage 4 sleep, the latter often being called delta sleep or slow wave sleep because of the slow brain waves occurring at this time known as delta waves. After 20-30 minutes of such slow wave sleep, the sleep pattern normally lightens into Stage 2 and then almost immediately changes into Stage 5 involving very active brain wave functioning and known as paradoxical or REM sleep. Stage 5 lasts 10-20 minutes before the sleep pattern "falls" back down into Stage 2 again.

This is the end of the first sleep cycle, following which a new sleep cycle begins. Gradually, the amount of delta sleep diminishes and is replaced with longer and longer periods of alternating Stage 2 baseline sleep and Stage 5 REM sleep. By the final sleep cycle of the night, approximately half of the cycle is spent in Stage 2 sleep and half in Stage 5 REM sleep.

The stages are described in greater detail below.

### Stage 1 Sleep

This is experienced as falling to sleep and is a transition stage between waking and sleeping. It usually lasts between 1 and 10 minutes. This stage is dramatically increased in some states of insomnia (restless legs) and in disorders that produce frequent arousals, such as sleep apnea and periodic limb movement in sleep (PLMS).

### Stage 2 Sleep

This follows Stage 1 sleep and is the "baseline" of sleep. This stage occupies approximately 50%-65% of sleep.

### Stage 3 and 4 Sleep

Stage 2 sleep evolves into "Delta" sleep or "slow wave" sleep in approximately 10-20 minutes and the slow wave sleep may last 20-30 minutes. It is called "slow wave" sleep because brain activity slows down dramatically from the "theta" rhythm of Stage 2 to a much slower rhythm of 1 to 2 cycles per second called "delta", and the height or amplitude of the brain waves increases dramatically. In most adults, these two Stages 3 and 4 are completed within the first two 90 minute sleep cycles or within the first three hours of sleep. Contrary to popular belief, it is delta sleep that is the "deepest" stage of sleep (not REM) and the most restorative. It is delta sleep that a sleep-deprived person's brain craves first and foremost. In children, delta sleep can occupy up to 40% of all sleep time, and this is what makes children unwakeable during most of the night.

### Stage 5 Sleep

REM (Rapid Eye Movement) sleep: This is a very active stage of sleep. It comprises 20-25 % of a normal night's sleep. Breathing, heart rate and brain wave activity quicken and vivid dreams can occur. Sleep specialists call this 5th stage of sleep "REM" (Rapid Eye Movement) sleep because the eyes of a person in this stage of sleep are moving rapidly to and fro. After the REM stage, the body usually returns to Stage 2 sleep.

The present invention, at least in its preferred form described below, makes use of a knowledge and monitoring of these five stages of sleep to provide a sleep enhancing device for assisting and enhancing the quality of sleep.

According to one aspect of the present invention, there is provided a sleep enhancing device comprising a transducer for detecting ambient noise in a sleeping environment and for generating electrical signals representing the ambient noise, means responsive to a state of sleep of an individual in the sleeping environment for providing a corresponding control input, a processor for analysing the electrical signals to evaluate the ambient noise and provide an evaluation output, a sound bank storing audio data representing soothing sounds, sound generating means responsive to the control input and the evaluation output for selecting audio data from the sound bank and providing an audio output, and output means for outputting the audio output as sound.

According to another aspect of the present invention, there is also provided a method of enhancing sleep comprising detecting ambient noise in a sleeping environment and generating electrical signals representing the ambient noise, providing a control input representing a state of sleep of an individual in the sleeping environment; analysing the electrical signals to evaluate the ambient noise; providing an evaluation output based on the evaluation; storing audio data representing soothing sounds; in response to the control input and the evaluation output, selecting audio data and providing an audio output; and outputting the audio output as sound.

The device is preferably designed to continue functioning throughout the night. Each night, or session, may comprise at least two modes: a pre-sleep, lullaby and noise masking, mode, and a sleep, noise masking, guardian mode. During each session, the progress of each mode is designed to follow the normal pattern of adult sleep through the five stages (as described above) from wakefulness to deep sleep.

In a first embodiment of the device, no data is available to the device as to the actual sleep state of the user and the stages of sleep are assumed and incorporated into logic programming for the device so that the durations of the pre-sleep mode and the sleep mode may be set by the user, using a suitable selection of pre-programmed presets.

For example, the pre-sleep and sleep modes may be determined by means of a timer pre-set by the user to allow a selected time period for each of the pre-sleep mode and at least one sleep mode.

Where only a single user is expected to be present in the sleeping environment and the breathing of the user is audible and thus likely to be detectable by the device, the sleep state may in addition, or instead, be determined on the basis of the detected breathing rate as picked up by the or a transducer of the device.

In another embodiment of the invention, means are provided for monitoring directly selected vital signs of the user to derive the state of sleep of the individual. For example, sensors may be employed, attached to the individual, for providing an output representing vital signs of the individual, such as temperature and heart rate, as well as further life signs, such as skin resistance, skin conductance or skin conductance change.

In such a case, the sensor information gathered by the device may be used to determine sleep state and anxiety level and thus to control:
(i) the mode of the device, which further determines the characteristics of the output sound, and
(ii) other relevant settings such as tempo, volume or other generative characteristics of the audio output.

In a preferred embodiment of the invention, at least some elements of the sleep enhancing device is incorporated in a user wearable device.

For example, the user wearable device may comprise a headband carrying sensors mounted so that in use they are placed in contact with the wearer's skin and headphones mounted in the area of the headband designed to cover the user's ears. The remainder of the electronics may be miniaturised and may be provided on a flexible circuit board connecting to the input and output elements, the whole assembly being incorporated within the fabric of the headband. Alternatively, only the sensors may be contained within the headband, and the controlling electronics may be contained within a separate unit, and connection between the headband and the separate unit may be made by a suitable wire or wireless data connection.

Alternatively, the user wearable device may comprise a soft, wrist-worn band carrying sensors mounted on the underside of the wrist-worn band so that in use they make contact with the wearer's skin. The necessary electronics may be miniaturised and provided on a flexible circuit board. As before, all the necessary components may be incorporated within the fabric of the wrist-worn band, or the electronics may be contained within a separate unit such that connection between the wrist-worn band and the separate unit of the device may be made by a suitable wire or wireless data connection.

Either such embodiment of the wearable sleep enhancing device according to the invention is preferably powered by primary battery cells, or rechargeable cells which can be easily recharged in between sessions or at any suitable interval from a suitable low voltage DC supply.

The invention will be described further, by way of example, with reference to the accompanying drawings, in which:
Figure 1 is a schematic block diagram representing a first embodiment of sleep enhancing device according to the present invention;
Figure 2 is a more detailed block diagram of the sleep enhancing device of Figure 1;
Figure 3 is a software flowchart representing the steps taken by a processor of the first embodiment of the device;
Figure 4 is a diagram representing a typical adult sleep cycle annotated with event cues and showing the response of the sleep enhancing device according to the first embodiment of the device;
Figure 5 is a perspective diagram of a second embodiment of sleep enhancing device according to the present invention, incorporated in a user wearable device in the form of a headband;
Figure 6 is a block diagram of the sleep enhancing device of Figure 5;
Figure 7 is a software flowchart representing the steps taken by a processor of the second embodiment of the device in order to detect and respond to sleep stage;
Figure 8 is a diagram showing the typical adult sleep cycle annotated with event cues and showing the response of the sleep enhancing device according to the second embodiment to certain sleep events ; and
Figure 9 is a perspective diagram of a third embodiment of the sleep enhancing device, employing a wrist-worn band incorporating a sensor array.

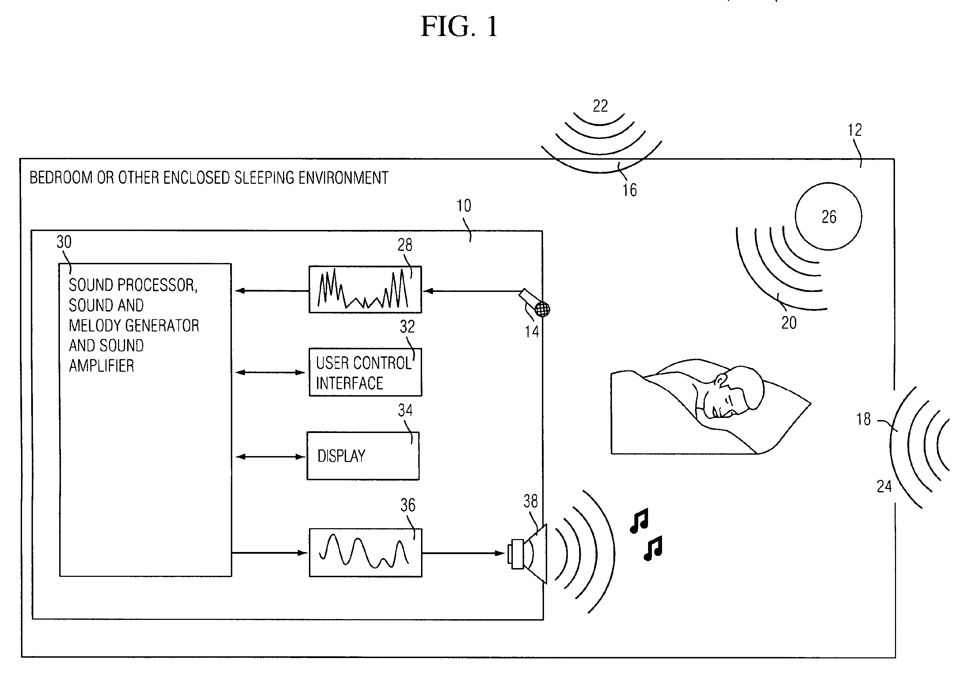

Referring initially to Figures 1 to 4, a first embodiment of sleep enhancing device according to the invention comprises a portable device 10 for use in a sleeping environment or room 12 of an adult person.

The device 10 has a microphone 14 for picking up a range of disturbing sounds 16, 18, 20 entering the sleeping environment from other rooms 22 within the building or through open doors or windows 24 or produced by a source 26 present within the sleeping environment 12, for example a partner or a mechanical device, such as an air conditioning unit. The microphone 14 serves for generating an analog audio signal represented in box 28 for supply to a sound processing unit 30. The sound processing unit 30 is controlled by means of a user control interface 32 operable, by means of manual knobs, dials, keys, proximity, voice or other conventional control means, to enable the user to set a functional state or control other settings of the sound processing unit 30. Such user set functional state and settings are indicated on a display 34, for example a light emitting diode display or a liquid crystal display. In dependence upon the set functional state and the nature and volume of the disturbing sounds, as well as on the stage within a sleeping session or on the individual's state of sleep, the sound processing unit 30 generates an analog output signal represented in box 36 for supply to a speaker 38 for output.

The sleep enhancing device as illustrated in Figure 1 is designed to detect and evaluate disturbing sounds in the sleeping environment, to adapt to the stage within a sleeping session of the individual in the sleeping environment, and to produce soothing sounds to reduce stress and/or to encourage or enhance a sleeping state in the individual and masking sounds to mask disturbing noises.

In a preferred embodiment as described below, the sound processing unit 30 incorporates a logic control circuit and software enabling the sleep enhancing device 10 to respond rapidly to disturbing noises in the sleeping environment. The sound processing unit 30 is capable of generating a range of tonal and atonal sounds and a range of volumes depending on the nature of the external noise present in the sleeping environment and on the relevant stage of sleep within a sleeping session, in order both to mask external noises in real time and to create a generative and evolving sound environment suitable for encouraging and/or enhancing sleep.

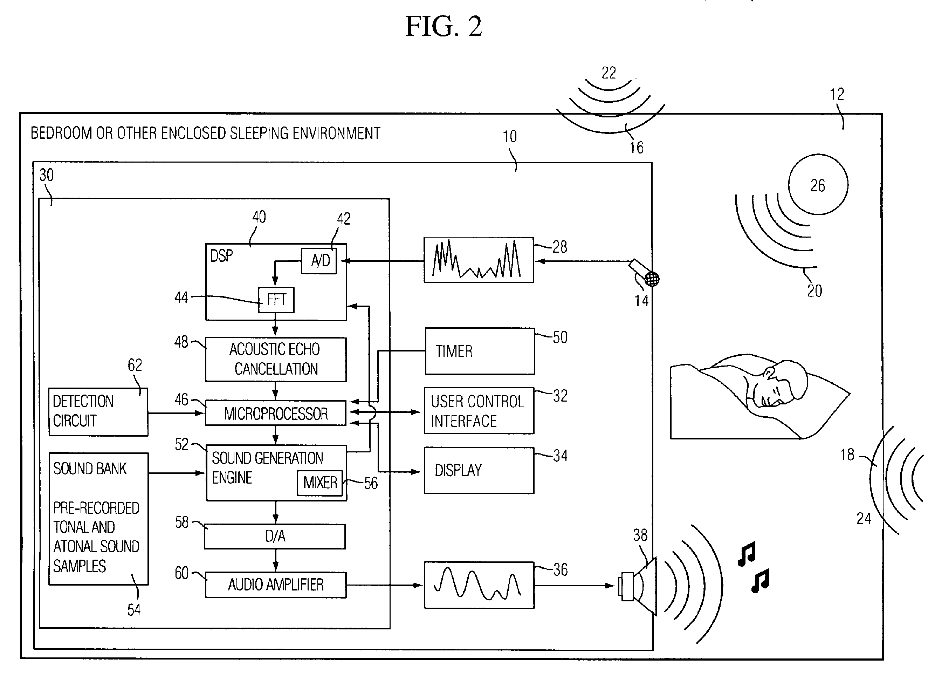

The sound processing unit 30 will now be described further with reference to Figure 2.

As shown in Figure 2, the sound processing unit 30 comprises a digital signal processor (DSP) 40 for receiving from the microphone 14 the analog audio signal 28 representing the ambient noise from the noise sources 22, 24, 26. The DSP 40 comprises an analog to digital converter 42, which receives the analog audio signal 28 and generates a data stream as output. The DSP 40 also comprises a fast fourier transform (FFT) analysis circuit 44 arranged to receive the data stream in order to analyse the input sound energy into a number of frequency bands for further analysis by a microprocessor 46, for example into 25 frequency bands corresponding to the 25 critical bands detectable by the human ear, as determined by known experimental data. For this purpose, the FFT circuit 44 providing a data analysis output from the DSP 40 is connected by way of an acoustic echo cancellation circuit 48 (to be described below) to the microprocessor 46, which is also connected to the user control interface 32 and display 34.

The microprocessor 46 monitors the data analysis output from the DSP 40 representing the analysed environmental noise and generates a corresponding control output. The microprocessor 46 also conducts a further analysis in order to detect the current stage of a sleeping session and determine the kind of sound output that would benefit the sleeper, and in response to this further analysis adapts the control output to trigger certain output sound modes, as described below. In the present embodiment, this further analysis is based on a timing output from a pre-set timer 50 representing a current stage in a sleeping cycle, the timer 50 being initialised at the start of operation of the sleep enhancing device 10 by user input from the user control interface 32.

The control output from the microprocessor 46 is supplied to a sound generation engine 52, which responds by generating a digital audio output representing a particular type of sound and which sets an output volume. For this purpose, the sound generation engine 52 refers to a sound bank 54 containing pre-recorded tonal and atonal sound samples stored in the sound bank 44 at the point of manufacture, and selects at least one of the sound samples for the sound output. For example, the atonal sounds may include short samples of filtered and modulated broadband "white-noise" and/or longer sound samples resembling gentle wind in trees or soft waves on a beach, and the tonal sounds may comprise multi-frequency samples suitable for synthesizing a large variety of musical sounds under instruction from the sound generation engine 52. In the embodiment of the invention shown in Figure 2, the sound generation engine 52 includes a mixer circuit 56 for mixing and blending a selected combination of various types of sound output generated by the engine by employing the sound samples of the sound bank 54.

The digital output from the sound generation engine 52 is supplied to a digital to analog converter 58 whose analog output is amplified in an audio amplifier 60 for driving the speaker 38 to produce the required sound in the human audible range. User adjustment of the manual controls on the user control interface 32 enables the user to control various characteristics of the sound output by the speaker 38 such as volume, type and range of sound, including the blend of tonal and atonal sound, tempo, harmonic density, output mode, and duration of sleeping session etc.

Further, in order to eliminate acoustic echo generated as a result of the microphone 14 detecting sound output by the speaker 38, a feedback connection is provided from the sound generation engine 52 to the DSP 40 and by way of the acoustic echo cancellation circuit 48 to the microprocessor 46.

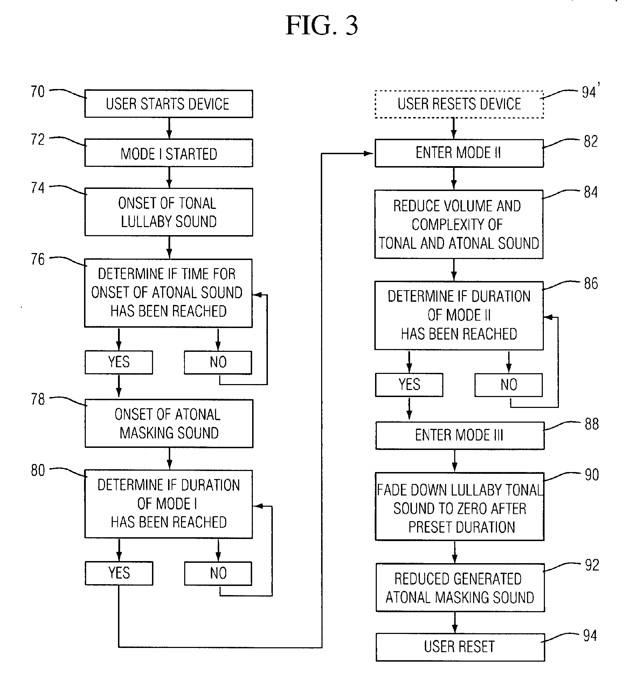

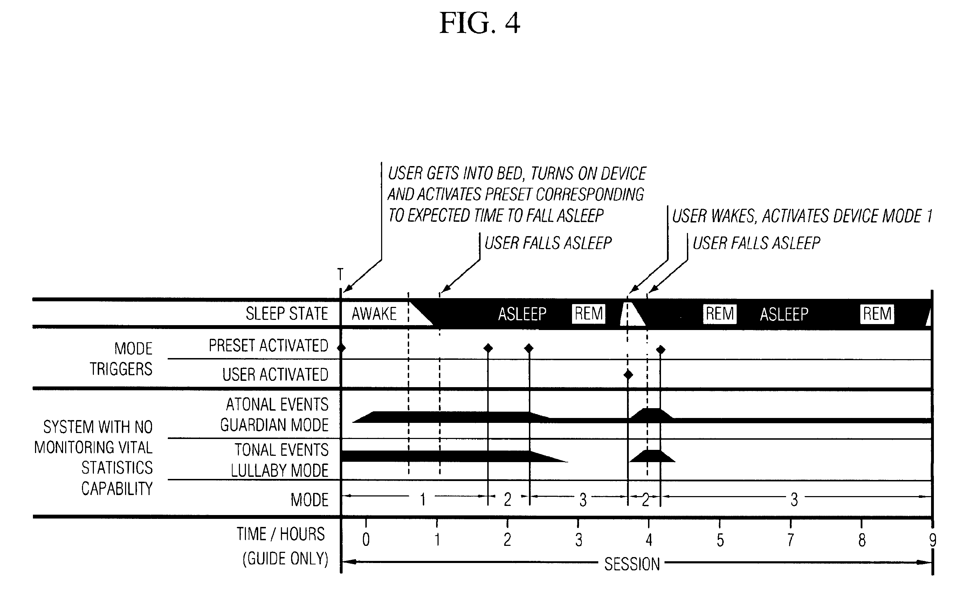

The sleep enhancing device 10 described above operates in three phases or modes, which are pre-set, or set by the user through the user control interface 32, and are subsequently monitored and triggered by the timer 50. A particular sleep session involving these three phases will now be described with reference to Figures 3 and 4, which show respectively a flow chart of the steps taken by the processor 30 and the various events occurring during the sleep session of the user. In Figure 4, the vertical axis represents the different concurrent states of: the user, the output of the timer 50, the user set input from the user control interface 32, the sound output of the device 10 and the mode of the device 10, and the horizontal axis represents the duration of the sleep session.

The sleep session starts when the user gets into bed and activates the device 10 in step 70 in Figure 3. This is shown as point T in Figure 4. Activation of the device 10 triggers a first phase (Mode 1, pre-sleep A) in step 72, in which the individual is considered to be awake. The microprocessor 46 commences in step 74 by causing the sound generation engine 52 to play a soft soothing blend of tonal sounds, notes and chords. During this sound output, the microprocessor 46 monitors the timer 50 to establish whether a predetermined time period has elapsed (step 76). If the answer is no, step 76 is repeated. After a predetermined time period determined by the timer 50, the answer to the enquiry of step 76 becomes yes, and the microprocessor 46 advances to step 78 and initiates the production of atonal sounds to supplement the tonal sounds and fulfil the function of noise masking in a way designed to relax the user. The atonal sounds output by the device 10 at this time may sound like gentle wind in trees or soft waves on a beach, and the tonal sounds may comprise musical phrasing of a gentle and calming lullaby nature. The tonal phrasing may typically occur on a single musical modality or scale where the progression is determined by a non-linear probabilistic algorithm designed to produce harmonically-related tonal sounds, generated in real-time by the sound generation engine 52. At some point during Mode 1, the user falls asleep, as shown in the uppermost line of Figure 4 where the clear band (denoting awake) is replaced by a shaded band (denoting asleep).

The microprocessor 46 continues to monitor the timer 50 in step 80 to determine whether another predetermined period representing the total duration of the Mode 1 has ended, and repeats step 80 until the timer 50 returns the answer yes. The microprocessor 46 then advances to step 82 and triggers a second phase (Mode 2, pre-sleep B), in which the individual is considered to be falling asleep. In practice, the user may already have fallen asleep as shown in Figure 4 but the timer 50 is preset to allow a fixed period for Mode 1. During Mode 2, the microprocessor 46 controls the sound generation engine 52 in step 84 to maintain the tonal output but such that this output becomes less musically complex. Atonal sounds are also maintained to mask any external noise, but the rhythm, dynamic range and overall amplitude level of the output of the device is gradually reduced in step 84.

The microprocessor 46 now monitors the timer 50 in step 86 to determine whether a further predetermined period representing the total duration of the Mode 2 has ended, and repeats step 86 until the timer 50 returns the answer yes. The microprocessor 46 then advances to step 88 and triggers a third phase (Mode 3, sleep), in which the individual is considered to be asleep or in the early stages of sleep. The tonal sounds are now reduced to zero in step 90 and the atonal sound masking is reduced in volume in step 92 to only that which is necessary to mask external noises. The third phase is maintained throughout the night until the individual wakes and resets the device 10 by means of the user control interface 32 in step 94.

If the user does wake during the night, the user can reset the device 10 to begin a new session or to return to any of the preceding phases. In the example given in Figures 3 and 4, the user resets the device 10 to the start of Mode 2, shown in Figure 3 as step 94' leading in to step 82. The microprocessor 46 now repeats the subsequent steps 84 to 92 as already described.

Thus, as shown in Figure 4, the device has a set duration for each mode, where the duration of each mode and the transitions between modes are the result of either user-determined settings via the user control interface 32 or internally predetermined factory-set presets. Settings chosen by the user may depend on their own personal experience and perception of how long they normally spend in each of the three modes of sleep. In cases where the user is experiencing severe insomnia and Mode 1 is anticipated to be longer than 30 minutes, the device 10 may also be set via the user control interface 32 into a training mode whereby the user determines a starting duration for Mode 1, but thereafter the device reduces this duration over a period of a number of days (for example between 14 and 28), with the intention of reducing the time it take the user to fall asleep.

In a variation of this embodiment, the timer 50 may be replaced, or supplemented, by a detection circuit 62 connected to and arranged to cooperate with the microprocessor 46 so as to detect breathing sounds received by the microphone 14 that originate from the sleeper. In this instance, the rate of breathing of the sleeper may be monitored throughout the night and the Mode and or sound output adjusted accordingly.

Where the breathing pattern is audible and measurable, the device 10 can modulate its function whilst in the during-sleep mode, such that noise masking is reduced to a minimum or even zero during stages of deep sleep, only increasing its function when the user is in REM sleep and more likely to become aware of external noise and to be disturbed or woken.

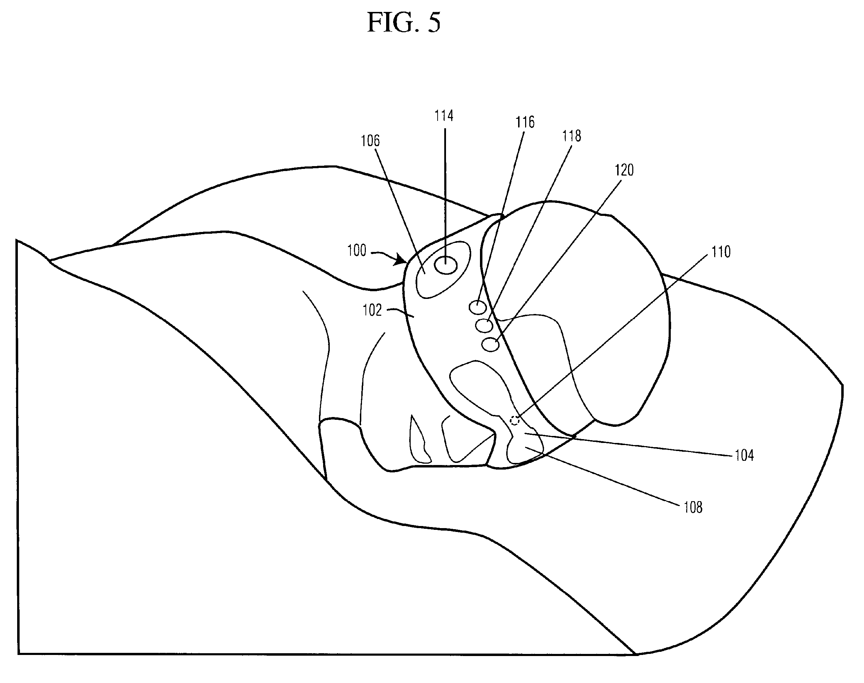

Referring now to Figures 5 to 8, a second embodiment of a sleep enhancing device 100 incorporated in a wearable headband will now be described. The sleep enhancing device 100 comprises many of the same features as the sleep enhancing device 10 of Figures 1 to 3, and like parts are designated by the same reference numerals and will not be described further.

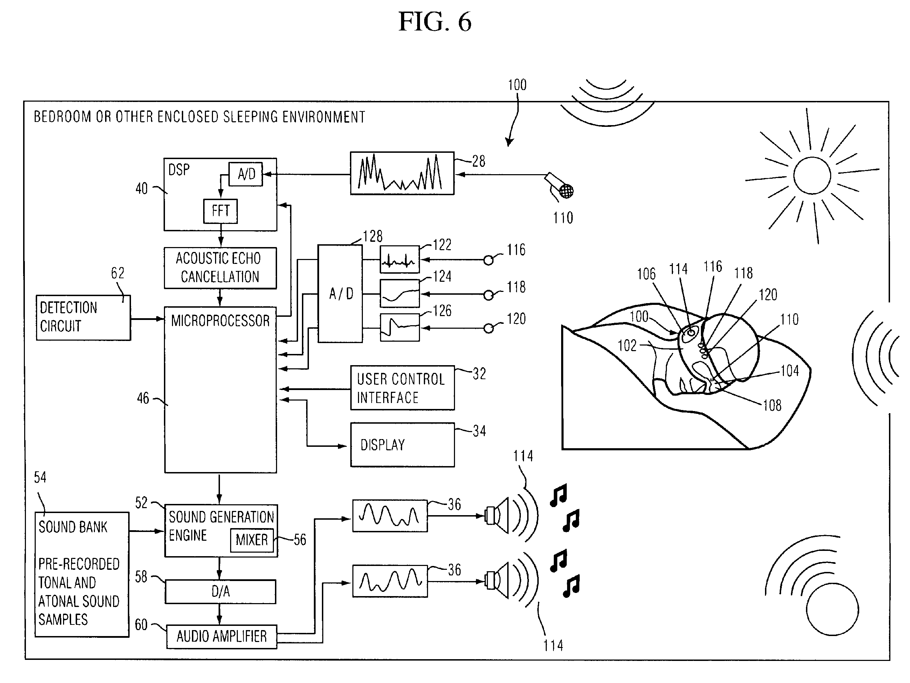

The sleep enhancing device 100 comprises a wearable headband 102, which is made from a soft, flexible material and which includes an eye masking portion 104 and an ear muffling portion 106. The eye masking portion 104 of the headband 102 incorporates an eye mask for reducing the levels of light falling on the individual's eyes, in order to reduce the possibility of the user being kept awake or woken by external light levels. Within the eye mask 104 is mounted a detector 110 corresponding to the microphone 14. Within the ear muffling portion 106 of the headband 102 is mounted a pair of stereo headphone speakers 114, only one being visible in the views of Figure 5 or 6.

In addition, the headband 102 carries a series of sensors 116, 118, 120 respectively for detecting and generating electrical signals representing heart rate, skin temperature and skin conductance. The sensors 116, 118 enable heart rate and skin temperature to be measured to determine the user's stage of sleep. Anxiety levels may also be monitored based on the detection of an increase in heart rate by the sensor 116 or the detection of gross changes in, or rapid variations in the rate of change of, skin conductance by the sensor 120.

The headband 102 also carries in miniature, the remaining electronics of the sleep enhancing device, for example mounted on a flexible printed circuit board 108, and a rechargeable battery cell for powering such electronics.

The processing circuits as shown in Figure 6 embedded within the headband 102 are similar to those of the device of Figure 2. In particular, the microphone 110 is connected to the DSP 40, which supplies signals to the microprocessor 46 as before. The microprocessor 46, in this instance, further receives analog signals representing the vital signs of the user from each of the sensors 116, 118 and 120, examples of such signals being shown respectively in boxes 122, 124 and 126. The analog signals are supplied by way of an analog to digital converter 128 to the microprocessor 46.

The microprocessor 46 evaluates the level and nature of the ambient noise in the sleeping environment 12 from the signals from the microphone 110 and the stage of sleep of the user from the signals derived from the sensors 116, 118 and 120, and determines the type of sound to be generated accordingly. The microprocessor 46 then produces an evaluation output for the sound generating engine 52, which supplies an audio output by way of the digital to analog converter 58 and the audio amplifier 60 to each of the headphone speakers 114. The sound output by the device is thus supplied directly to the user's ears and need not disturb a partner, if present.

An advantage of the present embodiment is that the user's stage of sleep may be closely monitored and tracked by means of the control input from the sensors 116, 118, 120 and separated into the five stages of sleep as described above. As a result, the three output modes described in relation to the first embodiment may be matched more closely to the sleeping state of the user or maybe refined by the addition of further output modes. In particular, it is known that heart rate reduces during the pre-sleep stage and skin temperature increases as the body prepares to go to sleep. In this embodiment, the microprocessor 46 can detect such transitions and generate corresponding control outputs for supply to the sound generating engine 52.

It is also possible by means of this embodiment to employ the data collected from the sensor 116 for detecting heart rate and from the sensor 120 for detecting skin conductance and deriving skin conductance change both to monitor the user's relative level of anxiety and determine whether the user is awake or asleep. For this purpose, the microprocessor 46 processes the detection signals and, where the user is considered to be experiencing a high level of anxiety (represented by a high level of skin conductance change and an elevated heart rate), slow gentle pulsing sounds, or other suitable calming sounds can then be output to help reduce anxiety.

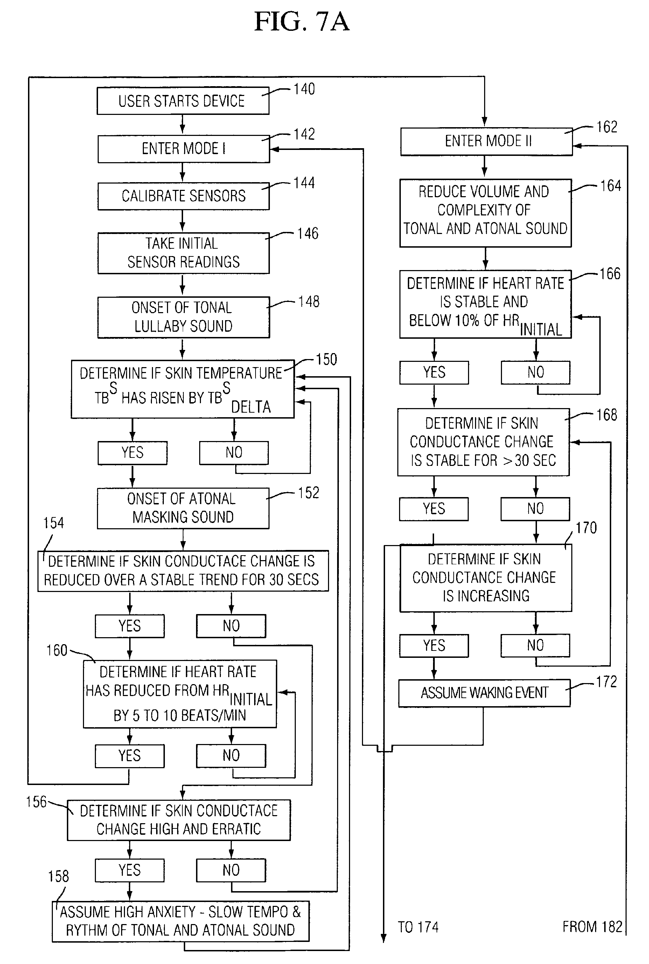

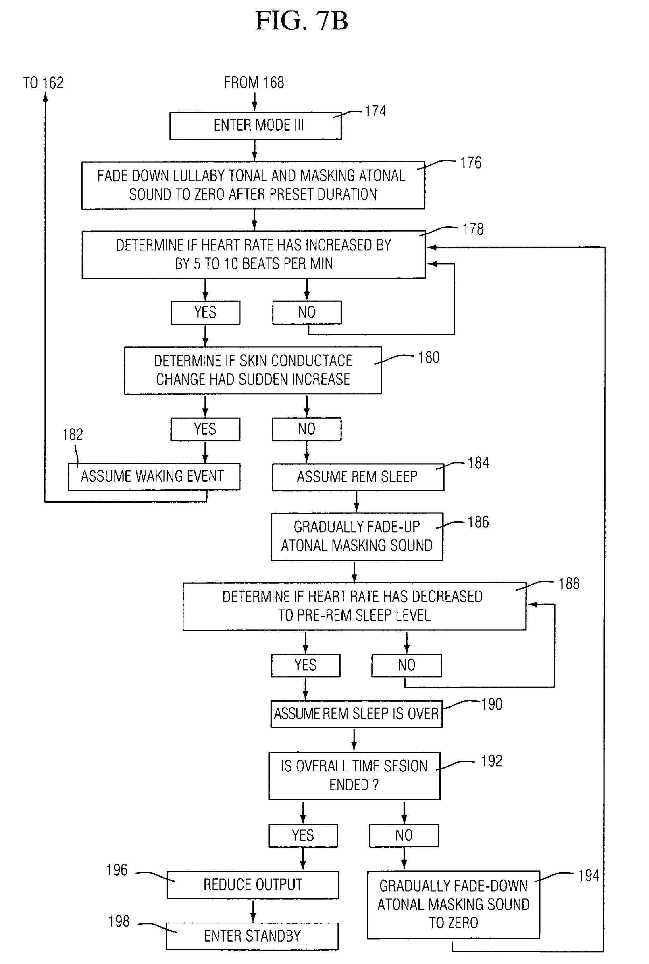

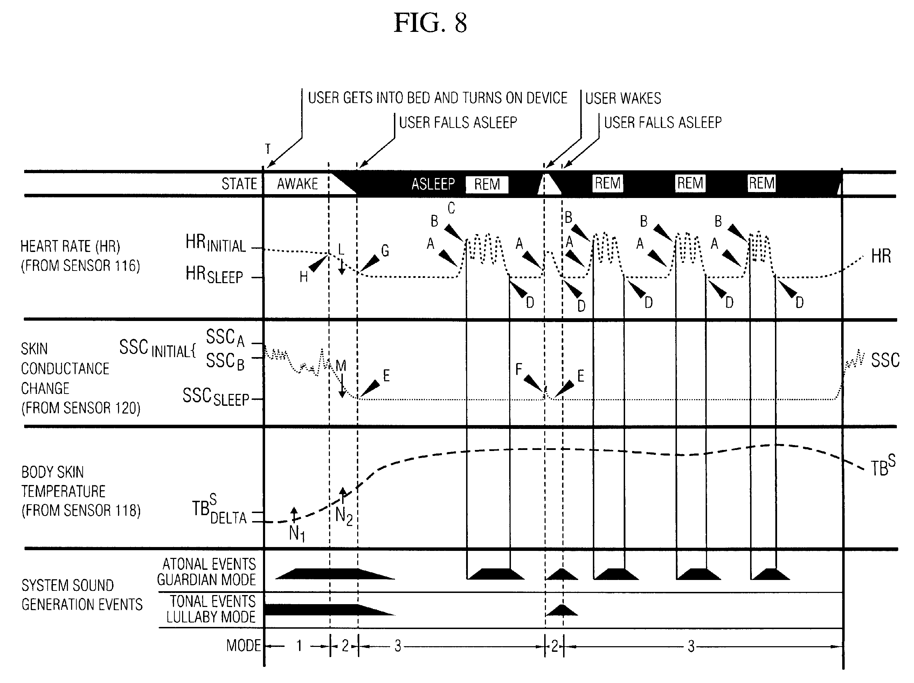

A particular sleep session will now be described with reference to Figures 7A, 7B and 8, which show respectively a flow chart of the steps taken by the processor 46 and the various events occurring during the sleep session of the user. In Figure 8, the vertical axis includes different bands representing respectively the system sound generation events, the different vital signs data recorded during a typical sleep session using sensors 116, 120, 118 (ie heart rate, skin conductance, and skin temperature respectively) and the user's sleep state, whereas, the relevant Mode of the device, the sound output of the device 10, the session duration and the sleep state of the user are all shown on the horizontal axis.

In this embodiment, as shown in Figure 8, the response of the sleep enhancing device 10 to certain sleep events is either timed or derived from sensor input, where the response of the device 10 is shown in terms of the output of atonal and tonal events generated following the analysis of vital signs to determine the device mode. For this purpose, heart rate (HR), skin conductance and hence skin conductance change (SCC), and skin temperature (TB^{s}) are all measured constantly by the skin sensors 116, 120, 118, as described above. The microprocessor 46 correlates the data from these three sensors to establish the physiological markers which allow the device 10 to determine accurately whether or not the user is awake or asleep and whether or not they are experiencing REM sleep (Stage 5 sleep).

The sleep session starts when the user gets into bed and activates the device 10 in step 140 in Figure 7A. This is shown as point T in Figure 8. Activation of the device 10 triggers a first phase (Mode 1, pre-sleep A) in step 142, in which the individual is considered to be awake. When the device 10 is initialised, it automatically calibrates the vital signs sensors 116, 118, 120 in step 144 and then takes initial measurement readings of heart rate, skin conductance change and skin temperature in step 146. The microprocessor 46 then commences in step 148 to cause the sound generation engine 52 to play an appropriate, soft soothing blend of tonal sounds, notes and chords.

Next, the microprocessor 46 checks the output of the sensor 118 in step 150 and enquires whether skin temperature has risen by an amount N₂ from the initial level TB^{S}_{DELTA}. Such a temperature rise during the early part of Mode 1 is a good indicator that the user's body is preparing for sleep. The microprocessor 46 keeps repeating step 150 until a temperature rise is detected, when the microprocessor 46 advances to step 152 and triggers the output of atonal masking sound.

The microprocessor 46 then checks the output from sensor 120 over a predetermined period of, say, 30 seconds in step 154 and enquires whether the skin conductance change is reducing in a stable manner, since skin conductance change is an accurate and robust measure of whether the user is awake or asleep. Skin conductance change, or absolute skin conductance may also be used to measure anxiety levels. Therefore, where no skin conductance decrease is detected, the microprocessor 46 determines in step 156 whether the skin conductance change is high and erratic. Where the skin conductance change is not high and erratic, the microprocessor reverts directly to step 150. However, where a high and erratic skin conductance change is detected, an elevated level of anxiety is assumed and the microprocessor 46 slows the tempo and rhythm of the sound generated by the sound generation engine 52 in step 158 before reverting to step 150. When the microprocessor 46 determines in step 154 that the skin conductance change is reducing, the microprocessor advances to step 160 and checks the output from the sensor 116 to establish whether there is a gradual reduction in heart rate, which taken in combination with a rise in skin temperature would signify that the user's body is relaxing and preparing for sleep. For example, the microprocessor 46 may check whether the initial heart rate HR_{INITIAL} has reduced by 5 to 10 beats per minute (a reduction of ~10% from a normal initial resting heart rate) for this purpose. The microprocessor 46 repeats step 160 until the requisite slowing of heart rate is noted, and then proceeds to step 162 at which point the device enters Mode 2.

Mode 1 (trying to fall asleep) can normally take anywhere from one minute to two hours. It is likely that users of the current invention will be in Mode 1 for at least 10 minutes, but this may be extended to a much longer period for people experiencing problems going to sleep. In particular, it is known that anxiety levels play a big part in the ability of the user to fall asleep and increase the possibility of noise disturbing the normal sleeping routine or the user's ability to fall off to sleep, all of which may affect the duration of Mode 1. A marked shift in the vital signs data is seen when the user actually starts to fall asleep; heart rate typically drops by 5 to 10 beats per minute, represented in Figure 8 by L, while skin conductance change rapidly decreases, represented by M, and body skin temperature continues to rise, represented by N₂. These events allow the device 10 to determine that the user is falling asleep and thus trigger the device 10 to enter Mode 2.

The process of falling asleep (Mode 2) can typically take between one minute and 20 minutes, or even longer in some cases. However, in cases where this takes longer than 20 minutes, the longer time is more usually due to a reversion to the previous state of trying to fall asleep.

At the start of Mode 2, the microprocessor 46 causes the sound generating engine 52 to reduce the volume and complexity of the atonal sounds and the tonal sounds in step 164. Next, the microprocessor 46 checks the output of the sensor 116 in step 166 to determine whether the heart rate is stable and below a predetermined percentage, eg ~10%, of HR_{INITIAL}. The microprocessor 46 repeats step 166 until a heart rate reduction of ~10% has been recorded and heart rate is stable, and then in step 168 checks the output from the sensor 120 to see if the skin conductance change has been stable for a predetermined period, e.g. 30 seconds. Where skin conductance change is not stable, the microprocessor 46 again in step 170 reviews the output from the sensor 120 to see if the skin conductance change is increasing. If not, the microprocessor reverts to step 168. However, if the skin conductance change is increasing, the microprocessor 46 advances to step 172 and assumes the user is waking up, and reverts to step 142 and Mode 1. On the other hand, if the outcome of step 168 indicates that the skin conductance change is stable, the microprocessor 46 advances to step 174 (see Figure 7B) and enters Mode 3. Thus, when the device 10 establishes that the heart rate and skin conductance change have both stabilised, as shown by G and E in Figure 8, the device 10 is triggered to enter Mode 3.

In Mode 3, the volume of both tonal lullaby and atonal masking sounds is slowly reduced to zero in step 176 in order to avoid abrupt changes in the sound levels around the sleeping user. All the vital signs continue to be measured by the sensors 116, 118, 120, and, in step 178, the microprocessor 46 determines whether the heart rate has increased by a given amount, eg 5 to 20 beats per minute. The microprocessor 46 repeats step 178 until the required increase is measured, at which point it advances to step 180 and checks whether there has also been a sudden increase in skin conductance change. If the outcome of step 180 is yes, the microprocessor 46 derives that there has been a concomitant increase in skin conductance change and heart rate and assumes that the user is waking in step 182 and reverts to step 162 and the start of Mode 2. If, however, the outcome of step 180 is that the skin conductance change is not increasing, the microprocessor 46 derives in step 184 that the user has begun their first period of Stage 5 Sleep (or REM sleep), which typically occurs approximately 90 minutes after falling asleep.

Stage 5, or REM sleep is characterised by an increase in heart rate (A to B in Figure 8) of typically between 8 and 10 beats per minute and the onset of an erratic heart rate (B to C). However, during this increased heart rate activity the skin conductance change and body skin temperature largely remain unchanged as shown in Figure 8. During REM sleep, the user is less deeply asleep and may be more easily disturbed and woken by external noises impinging into their surrounding environment. Thus, when the device detects that the user is in REM (Stage 5) sleep, as described above, it remains in Mode 3 but gently increases the volume of the noise masking sound (atonal component) in step 186 in order to provide a stable sound environment and help the user to continue sleeping undisturbed even where there are sudden or fluctuating external noises.

After a period, typically of 30 minutes, the user generally moves out of REM sleep, reverting to a deeper sleep state once again, a transition which will be marked by a drop in the user's heart rate, back to the sleeping value (D in Figure 8) and its stabilisation at this resting level. Thus, in step 188, the microprocessor 46 evaluates the output of the sensor 116 to determine whether the heart rate has decreased to the pre-REM resting sleep level. The microprocessor 46 repeats step 188 until a reduced and stable heart rate is measured, at which point the microprocessor 46 advances to step 190 and assumes that the REM sleep is over. The microprocessor 46 then checks in step 192 whether the overall sleep session duration is ended (i.e. it is time to wake up) on the basis of the original settings input by the user, using the control interface 32. If the session is not at an end, the microprocessor 46 slowly causes the sound generating engine 52 to reduce the volume of the masking atonal sounds to zero in step 194, thus matching the sound output to the decrease in heart rate, and then reverts to step 178 and continues to monitor heart rate to determine when the next REM sleep cycle is commencing. This REM cycle is expected to be repeated a number of times each night, and each time the device 10 reverts to step 178 and follows the cycle, gradually fading the masking output up at the start of each REM cycle and then fading the masking output down at the end of each REM cycle. When the overall sleep session duration as set by the user is at an end, as determined in step 192, the microprocessor 46 proceeds to step 196 and reduces all outputs to zero and then in step 198 enters standby mode, from which it may only be reactivated by intervention by the user via the user control interface 32.

Referring to Figure 8, if at any time during the cycle an increase in heart rate A is accompanied by a similar increase in skin conductance change F, then the device 10 determines that the user has woken, or is in the process of waking. If this occurs before the user-determined setting for the end of the total session duration has been reached, this is assumed to be an undesirable waking event and the device 10 automatically reverts to Mode 2, and both atonal noise masking and tonal elements are gently faded up in volume. Mode 2 is maintained for the duration of the waking event until the microprocessor 46 determines that the heart rate has returned to the stable sleeping level D and the skin conductance change has stabilised at the sleeping level E. When the device 10 reaches the end of the user-determined session duration, typically six to eight hours, the microprocessor 46 reduces all outputs to zero and enters standby mode.

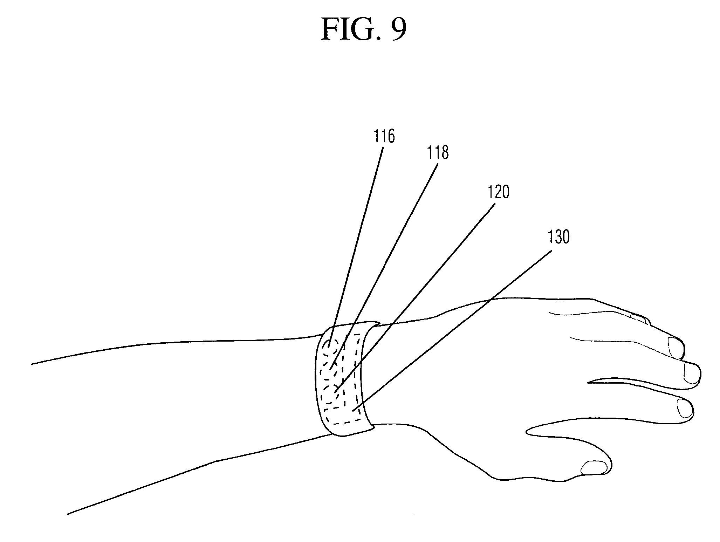

In a variation of the second embodiment shown in Figure 9, the headband 102 is replaced by a wristband 200 incorporating the sensors 116, 118 and 120 and a data transfer device 130, such as a wireless communication device, for transmitting the outputs from the sensors to the remainder of the sleep enhancing device as shown in Figure 6. The wristband 200 may be employed with or without a separate headband containing an eye mask and headphone output facility. In this embodiment, the wristband is thus preferably only a means for collecting the various vital sign information and providing data communication to a base unit, and the microphone input, speaker output and sound generative electronics are all contained within a base unit.

A significant advantage of using a wearable device, such as the headband or wristband of the second and third embodiments of the invention, is that it facilitates the non-invasive, passive measurement and monitoring of various aspects of the user's physical state vital signs, such as temperature, heart rate and skin conductance, by means of self-calibrating sensors place in direct contact with the user's skin. In addition, in the case of the headband, the stereo headphones ensure that the sound output of the device is limited solely to the user and provide an audio attenuation of external noise. At the same time, the eye mask in this embodiment reduces light levels falling on the user's eyes.

Various other modifications are also possible within the scope of the present invention.

For example, in another variation of the second embodiment of the sleep enhancing device 100, the electronics instead of being mounted within the headband 102 are separate and are connected by flexible wiring to the sensors and output devices within the headband 102. Such wired connection may, or course, alternatively be replaced by a wireless data connection.

Further, while the second and third embodiments above provide sensors for measuring respectively heart rate, skin temperature and skin resistance, it is equally possible instead or as well to further sensors for measuring other physiological signs.

## Claims

1. A sleep enhancing device comprising:
a transducer for detecting ambient noise in a sleeping environment and for generating electrical signals representing the ambient noise;
means responsive to a state of sleep of a user in the sleeping environment for providing a corresponding control input;
a processor for analysing the electrical signals to evaluate the ambient noise and provide an evaluation output;
a sound bank storing audio data representing soothing sounds;
sound generating means responsive to the control input and the evaluation output for selecting audio data from the sound bank and providing an audio output; and
output means for outputting the audio output as a sound output.

2. A sleep enhancing device according to claim 1 in which the sound generating means is arranged in response to the control input and the evaluation output to provide an audio output representing at least one of a melodic sound and a masking sound.

3. A sleep enhancing device according to claim 1 or 2 in which the processor controls the sound generating means to produce at least two modes of sound output.

4. A sleep enhancing device according to claim 3 in which the sleep state responsive means comprises a timer for controlling the duration of each of the at least two modes.

5. A sleep enhancing device according to claim 3 or 4 further comprising a user control interface for setting parameters of the at least two modes.

6. A sleep enhancing device according to any preceding claim further comprising detection means adapted to cooperate with the processor to distinguish the breathing rate of the individual.

7. A sleep enhancing device according to any preceding claim in which the sleep state responsive means comprises at least one sensor for detecting a physiological condition of the individual.

8. A sleep enhancing device according to claim 7 in which the at least one sensor detects at least one of the physiological conditions including temperature, heart rate, skin resistance and skin conductance.

9. A sleep enhancing device according to any preceding claim in which the processor analyses the electrical signals into frequency bands for monitoring the ambient noise.

10. A sleep enhancing device according to any preceding claim in which the processor analyses the electrical signals to monitor changes in the ambient noise.

11. A sleep enhancing device according to any preceding claim in which the sound bank stores pre-recorded tonal samples.

12. A sleep enhancing device according to any preceding claim in which the sound bank stores pre-recorded atonal sound samples.

13. A sleep enhancing device according to any preceding claim in which the transducer comprises a microphone.

14. A sleep enhancing device according to any preceding claim in which the output means comprises a speaker.

15. A sleep enhancing device according to any preceding claim at least partially incorporated in a user wearable device.

16. A sleep enhancing device according to claim 15 in which the user wearable device is a headband.

17. A sleep enhancing device according to claim 16 in which the output means comprise headphones mounted in the headband.

18. A sleep enhancing device according to claim 15 in which the user wearable device is a wristband.

19. A method of enhancing sleep comprising:
detecting ambient noise in a sleeping environment and generating electrical signals representing the ambient noise;
providing a control input representing a state of sleep of a user in the sleeping environment;
analysing the electrical signals to evaluate the ambient noise;
providing an evaluation output based on the evaluation;
storing audio data representing soothing sounds;
in response to the control input and the evaluation output selecting audio data and providing an audio output; and
outputting the audio output as sound.
